(19) Europäisches Patentamt
European Patent Office
Office européen des brevets

(11) Veröffentlichungsnummer: **0 330 745 A2**

(12) **EUROPÄISCHE PATENTANMELDUNG**

(21) Anmeldenummer: **88120249.3**

(51) Int. Cl.4: **C07C 49/713 , C07C 45/64**

(22) Anmeldetag: **05.12.88**

(30) Priorität: **03.03.88 DE 3806835**

(43) Veröffentlichungstag der Anmeldung: **06.09.89 Patentblatt 89/36**

(84) Benannte Vertragsstaaten: **CH DE FR GB IT LI NL**

(71) Anmelder: **HÜLS AKTIENGESELLSCHAFT**
**Patentabteilung / PB 15 - Postfach 13 20**
**D-4370 Marl 1(DE)**

(72) Erfinder: **Bellut, Hans, Dr.**
**Bergstrasse 58**
**D-4408 Dülmen(DE)**

(54) **Verfahren zur Herstellung von 3,5,5-Trimethyl-4-hydroxi-2-cyclohexen-1-on.**

(57) Es wurde nach einem wenig aufwendigen Weg zur Herstellung von 3,5,5-Trimethyl-4-hydroxi-2-cyclohexen-1-on durch Oxidation von beta-Isophoron gesucht. Bekannte Verfahren mit Edelmetallkatalysatoren, Peressigsäure oder m-Chlorbenzoesäure sind aufwendig in der Isolierung oder zeigen schlechte Ausbeuten.

Das neue Verfahren zur Herstellung von 3,5,5-Trimethyl-4-hydroxi-2-cyclohexen-1-on besteht darin, daß beta-Isophoron in Gegenwart von Perhydrol und Ameisensäure oxidiert wird.

3,5,5-Trimethyl-4-hydroxi-2-cyclohexen-1-on wird als Aroma- und Duftstoff verwendet.

EP 0 330 745 A2

## Verfahren zur Herstellung von 3,5,5-Trimethyl-4-hydroxi-2-cyclohexen-1-on

3,5,5-Trimethyl-4-hydroxi-2-cyclohexen-1-on (4-Hydroxi-isophoron) wird in der Literatur als Aromastoff für Tabakerzeugnisse (JP-OS 81 35 990; CH-PS 549 961; DE-OS 22 02 066) als Aroma- und Duftstoff in Lebensmitteln (CH-PS 549 956; M. Ishihara et al., J. Org. Chem. 1986, 51, 491-5) sowie als Grundstoff für die Synthese verschiedener Pharmazeutika (N. S. Zarghami et al., Phytochemistry 1971, 10, 2755-61; J. N. Marx und F. Sondheimer, Tetrahedron, Suppl. No. 8, Pt 1, 1-7, 1966) beschrieben. Die Synthese erfolgt regelmäßig durch Oxidation von beta-Isophoron (3,5,5-Trimethyl-3-cyclohexen-1-on) nach verschiedenen Methoden.

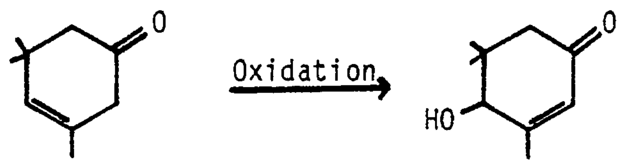


Da aber bis vor kurzem ein Engpaß bereits darin bestand, daß kein praktikables Verfahren zur Herstellung von beta-Isophoron bekannt war, wurden weiterführende Synthesemöglichkeiten erst gar nicht erforscht. Gemäß DE-OS 37 35 211 läßt sich aber nun bequem beta-Isophoron aus alpha-Isophoron (3,5,5-Trimethyl-2-cyclohexen-1-on) durch katalytische Isomerisierung herstellen, so daß zumindest dieser Engpaß beseitigt ist.

Durch die Oxidation in p-Stellung zur Carbonylgruppe wird im Isophoronmolekül ein reaktionsfähiger Substituent erhalten, der weitere Synthesen zur Gewinnung naturidentischer Riech- und Aromastoffe sowie zur Herstellung von Vitamin-A-Derivaten ermöglicht. Es wurde deshalb nach einem möglichst wenig aufwendigen und von der Chemikalienseite kostengünstigen Weg gesucht, diesen Oxidationsschritt auszuführen. Dabei ist zu berücksichtigen, daß beta-Isophoron sich leicht zu alpha-Isophoron umlagert, welches die gewünschte Reaktion nicht eingeht und damit zunächst für die Gewinnung von 4-Hydroxi-isophoron verloren ist. Es mußte also ein Weg gefunden werden, der zusätzlich zu den genannten Bedingungen die Rückisomerisierung in Grenzen hält.

Es ist bekannt, daß beta-Isophoron an Edelmetallkontakten mit Luft oxidierbar ist (FR-A 2 335 486); dabei wird neben 4-Ketoisophoron und alpha-Isophoron auch das gewünschte 4-Hydroxiisophoron als Nebenprodukt erhalten. In geringen Ausbeuten läßt sich auf biochemischem Wege mit Aspergillus niger 4-Hydroxi-isophoron sogar direkt aus alpha-Isophoron gewinnen (JP-OS 81 35 990; Y. Mikami et al., Agric. Biol. Chem. 1981, 45, 791-3). A. Heymes und P. Teisseire erhalten durch Oxidation mit Monoperphthalsäure in 34 % Ausbeute 4-Hydroxiisophoron (Recherches 1971, 18, 104-8), während N. S. Zarghami und D. E. Heinz (Phytochemistry 1971, 10, 2755-61) bei der Umsetzung mit Peressigsäure keine Ausbeuteangaben offenbaren. J. N. Marx und F. Sondheimer (Tetrahedron, Suppl. No. 8, Pt 1, 1-7, 1966); vgl. auch O. Isler et al., Helv. Chim. Acta 39, 2041, 1956) berichten über eine Ausbeute von 87 % beim Behandeln von beta-Isophoron mit m-Chlorperbenzoesäure, jedoch ergibt die Nachrechnung ihrer mitgeteilten Ergebnisse lediglich eine Ausbeute von 56 %, was auch mit anderen, früheren Literaturangaben übereinstimmt.

Alle bisher veröffentlichten Methoden weisen gravierende Mängel auf. Zum einen sind die Ausbeuten schlecht, die verwendeten Chemikalien teuer und die Isolierung des Endproduktes umständlich und kostenaufwendig; zum anderen werden die Verfahren mit großen Abfallmengen belastet. Ein technisches Verfahren auf Basis der vorliegenden Literatur ist so mit ziemlicher Sicherheit auszuschließen.

Es wurde deshalb ein neues Verfahren gesucht, das einfach und mit billigen Hilfsstoffen durchzuführen ist. Dabei wurde überraschenderweise gefunden, daß die Oxidation von beta-Isophoron zu 4-Hydroxi-isophoron mit handelsüblichem Perhydrol gelingt.

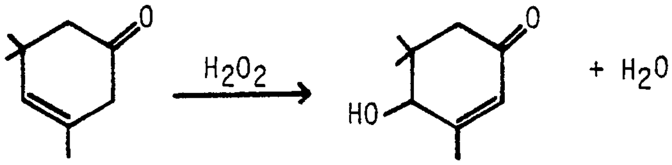


Gegenstand der Erfindung ist daher ein Verfahren zur Herstellung von 3,5,5-Trimethyl-4-hydroxi-2-cyclohexen-1-on durch Oxidation von beta-Isophoron, welches dadurch gekennzeichnet ist, daß die Oxidation in Gegenwart von Perhydrol und Ameisensäure bei Temperaturen zwischen 0 und 100 °C erfolgt.

Die Perhydrolkonzentration kann 25 bis 45 % betragen.

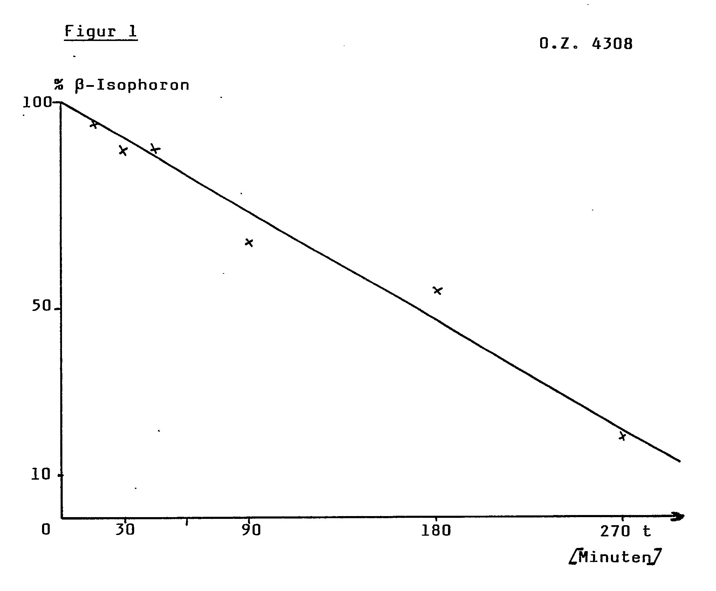

Um die bekannte Oxiranbildung bei dieser Reaktion zu verhindern, wird vorteilhaft in saurem Medium, insbesondere im ameisensaurem Medium, gearbeitet. Andere Säuren, besonders anorganische, katalysieren die Rückisomerisierung des beta-Isophorons im besonderen Maße, während bei Verwendung der weniger aggressiven Ameisensäure genügend Zeit für die oxidative Umsetzung des größten Teils an beta-Isophoron verbleibt. Das in Figur 1 gezeigte Diagramm zeigt den zeitlichen Verlauf der Rückbildung von alpha-Isophoron in einer Ausgangsmischung molarer Mengen beta-Isophoron und Ameisensäure bei Raumtemperatur.

Organische Säuren haben darüber hinaus den Vorteil der besseren Löslichkeit für alle Reaktionsteilnehmer. Ein weiterer Vorteil für die Verwendung von Ameisensäure liegt darin begründet, daß ein Überschuß an Perhydrol bei der anschließenden Aufarbeitung sicher vernichtet wird und keine Gefahr spontaner Peroxidzersetzung besteht.

$H_2O_2 + HCOOH \rightarrow CO_2 + 2\ H_2O$

Die Reaktion wird so geführt, daß gegen Ende der Umsetzung die Bedingungen für die sichere Vernichtung überschüssigen Perhydrols eingestellt werden. Das Reaktionsprodukt kann nach Neutralisation als organische Phase abgetrennt und destilliert werden.

Das hier beschriebene Verfahren gestaltet sich technisch relativ einfach. Als Hilfsstoffe werden billige, handelsübliche Chemikalien eingesetzt. In einem Temperaturbereich zwischen 0 °C und 100 °C werden 30 %iges Perhydrol und Ameisensäure im Molverhältnis 1 : 1 bis 5 : 1 in einer Rührapparatur vorgelegt und mit 0,5 bis 2,5 Mol, insbesondere 1 Mol beta-Isophoron langsam versetzt. Je nach vorgewählten Temperaturbedingungen und Ansatzgröße muß die Reaktionswärme durch leichte Kühlung abgeführt werden. Nach Beendigung der Nachreaktion und weiteren 12 Stunden Stehenlassen, wird der homogen gewordene Ansatz mit Hydrogencarbonat neutralisiert, die entstehende obere Phase abgetrennt, getrocknet und im Vakuum destilliert. Nach Abtrennung von rückisomerisiertem alpha-Isophoron wird 4-Hydroxi-isophoron als Destillat erhalten. Das im Vorlauf abdestillierte alpha-Isophoron kann nach erneuter Isomerisierung gemäß DE-OS 37 35 211 wieder in den Prozeß zurückgeführt werden.

## Beispiel 1

120 g (2 mol) 30 %iges Perhydrol werden mit 48 g (1 mol) Ameisensäure vermischt und bei 40 °C mit 138 g (1 mol) beta-Isophoron in 1/2 Stunde unter Rühren versetzt. Man läßt bei 40 °C (leichte Außenkühlung und Rühren) nachreagieren, um nach 12stündigem Stehenlassen kurzzeitig (1/2 Stunde) auf 80 °C aufzuheizen. Im Anschluß daran wird mit Natriumbicarbonat neutralisiert, die entstehende obere Schicht abgetrennt, mit Natriumsulfat getrocknet und im Vakuum destilliert.

Es werden bei

$Kp_4$ = 68 - 92 °C 44 g entsprechend 32 % alpha-Isophoron und

$Kp_{0,2}$ = 124 °C 72 g entsprechend 47 % 4-Hydroxi-isophoron

mit $n_D^{20}$ = 1,5010

erhalten.

Berücksichtigt man den Umsatz und das zurückerhaltene alpha-Isophoron, so beträgt die Ausbeute 69 % Produkt.

## Beispiel 2 und 3

In analoger Weise wie im Beispiel 1 werden Beispiel 2 und 3 durchgeführt, nur wird als Reaktionstemperatur 60 °C (Beispiel 2) bzw. 80 °C (Beispiel 3) gewählt. Die erzielten Produktmengen betragen:

Beispiel 2:
42 g entsprechend 31 % alpha-Isophoron und
59 g entsprechend 39 % 4-Hydroxi-isophoron
Die Ausbeute bezogen auf den Umsatz beträgt 66 %

Beispiel 3:
46 g entsprechend 34 % alpha-Isophoron und
63 g entsprechend 42 % 4-Hydroxi-isophoron
Die Ausbeute bezogen auf den Umsatz beträgt 70 %.

Beispiel 4

Es wird analog Beispiel 1 verfahren, doch wird die verwendete Menge Perhydrol soweit erhöht, daß ein Molverhältnis von 3 : 1 Perhydrol : beta-Isophoron eingestellt wird. Die erzielten Produktmengen sind dann:
27 g entsprechend 20 % alpha-Isophoron und
85 g entsprechend 56 % 4-Hydroxi-isophoron.
Die Ausbeute bezogen auf den Umsatz errechnet sich zu 82 %.

## Ansprüche

1. Verfahren zur Herstellung von 3,5,5-Trimethyl-4-hydroxy-2-cyclohexen-1-on durch Oxidation von beta-Isophoron,
dadurch gekennzeichnet,
daß die Oxidation in Gegenwart von Perhydrol und Ameisensäure bei Temperaturen zwischen 0 und 100 °C erfolgt.

2. Verfahren nach Anspruch 1,
dadurch gekennzeichnet,
daß Perhydrol in einer wäßrigen Lösung mit 25 bis 45 % Perhydrol, insbesondere mit 30 %, eingesetzt wird.

3. Verfahren nach Anspruch 1,
dadurch gekennzeichnet,
daß 30 %iges Perhydrol mit Ameisensäure im Molverhältnis 1 : 1 bis 5 : 1 vorgelegt und mit 0,5 bis 2,5 Mol beta-Isophoron, insbesonders 1 Mol, versetzt wird.

Figur 1

O.Z. 4308